# EUROPEAN PATENT APPLICATION

(11) **EP 4 424 295 A1**
(43) Date of publication of application: **04.09.2024**
(21) Application number: 23175407.8
(22) Date of filing: 25.05.2023
(51) Int. Cl.: A61K 8/00, A61Q 19/02, A61K 36/18, A61P 37/00, A23L 33/105, A61Q 19/08

(54) **PREPARATION METHOD OF A RICE BRAN EXTRACT COMPOSITION AND ITS USE FOR SKIN CARE**

(30) Priority: 03.03.2023 TW 112107722
(71) Applicant: CPC Corporation, Taiwan, Kaohsiung City 811251 (TW)
(72) Inventor: Chen, Jui-Hui, Chia-Yi City (TW); Jhah, Yu-Sin, Chia-Yi City (TW); Lin, Shou-Lin, Chia-Yi City (TW); Luo, Sheng-Jen, Chia-Yi City (TW); Chen, Chin-Chung, Chia-Yi City (TW); Chen, Chin-Kun, Chia-Yi City (TW); Wang, Yih-Ping, Chia-Yi City (TW); Tsai, Ming-Chang, Chia-Yi City (TW)
(74) Representative: Cabinet Chaillot

(57) **Abstract**

The present invention provides a preparation method of a rice bran extract composition and its use for skin care wherein the preparation method comprises steps as follows: rice bran and a solvent with a concentration more than 80% are mixed for development of a mixed solution and stirred as well as extracted at 40-55°C; the mixed solution is centrifuged or filtered for collections of supernatants or filtrates; the supernatants or filtrates go through a vacuum concentration process for collections of rice bran extracts; the rice bran extracts are treated for preparation of a rice bran extract composition. As shown in the present disclosure, rice bran rich in healthcare elements but usually abandoned are recycled and manufactured as a rice bran extract composition that is added into a personal skin-care product for promotion of skin whitening, anti-senescence, anti-inflammation and anti-allergy and a solvent treated in a vacuum concentration process is also recycled and reused as the solvent during preparation of the present invention for resources economization and efficiency of recycling economy.

## Description

### BACKGROUND OF THE INVENTION

### FIELD OF THE INVENTION

The present invention relates to a preparation method of a rice bran extract composition and its use for skin care, particularly effects for skin whitening, anti-senescence, anti-inflammation and anti-allergy and applications in skin care.

### DESCRIPTION OF THE PRIOR ART

Rice bran as one product of rice milling during which brown rice is transferred to white rice contains 12~16% protein, 16~22% grease, 20~25% dietary fibers, lots of trace minerals and phytochemicals such as γ-oryzanol, phytosterol, vitamin E, squalene and glucosylceramide. However, rice bran is hard to keep fresh but rotten easily and is unpopular with ordinary consumers due to grains coarse. Only few rice bran is deactivated or roasted before eating and selling by a handful of farmers for organic farming. In the past, rice bran is taken as fodders or even wastes to be abandoned directly that aggravate environmental burden definitely. As disclosed in many literatures, phytochemicals in rice bran have been critically acclaimed for the effect of skin health such that rice bran extracts are added into products for promotion of their effects and values. However, rice bran is a common material and easily available, when there is no extraction method or detailed final extract state, its extract is an ambiguous object with questionable both reproducibility of efficacy and convenience of use.

For an application of extracting rice bran in a known method, rice bran should be treated with hot water 6~10 times greater than rice bran in volume at more than 75°C first. With temperature cooled down to less than 40°C, rice bran is extracted through ethanol (concentration less than 80%) for at least 30 minutes after which first supernatants are centrifuged or first filtrates are filtered. The obtained extract (supernatants or filtrates) can then be used immediately. Alternatively, the obtained extract goes through a further vacuum concentration process to get separated supernatants and precipitates. Next, the precipitates are treated with aqueous alcohols (methanol, ethanol or propanol) to obtain second supernatants and precipitates. However, in addition to above cumbersome steps, when non-defatted rice bran is involved, the toxic solvent, n-hexane, is commonly used during spray drying of the first supernatants.

In another known method, an extraction solution in which methanol (acetone or ethanol) and water are mixed in the ratio of 30~70:70~30 (v/v) is prepared as a solvent (concentration ranging from 30% to 70%) for extraction based on the weight percentage of 1 (rice bran): 1~20 (extraction solution). With temperature set to 50~70°C, the extraction lasts for one to three hours. The in-vitro biochemical tests should be applicable to extracts. In some other known method, rice bran is treated with enzymes and extracted through a solution containing water and ethanol (the ratio of water to ethanol ranging from 1:1 to 25:1, i.e., 50%~3.8% ethanol) for six hours to seven days at temperature between 4 and 40°C.

As shown in above-mentioned techniques, a process for extraction of rice bran may show varying difficulties, for example, rice bran defatted or non-defatted, type/concentration of a solvent, use or not of an enzyme and temperature/time for extraction. Moreover, discrepancy exists in a functional test such as in vitro testing or in vivo cell assay. Without marker constituents, it is difficult to compare the efficacy of different methods, or even compare the batches when the extracts are comes from same method.

Furthermore, phytochemicals in rice bran such as γ-oryzanol, phytosterol, vitamin E, squalene and glucosylceramide for promotion of skin health are oil-soluble substances mostly which are hard to be extracted through a low-concentration solvent. As shown in one known method, high temperature contributes to extraction of oil-soluble substances, for example, rice bran is first treated with hot water at temperature more than 75°C and cooled down to less than 40°C at which extraction through a solvent is conducted. In another known method, temperature for extraction increases to 50~70°C but the concentration of a solvent is kept at less than 70%. In a further known method, oil-soluble substances are extracted through a low-concentration (<50%) solvent by means of enzymes and longer time for extraction. However, these known methods feature steps troublesome and much time wasted, for example, two steps including water treatment and solvent treatment and longer time spending in extraction. Despite high temperature favorable to extraction, oil-soluble substances damaged or oxidized are attributed to high-temperature treatment.

### SUMMARY OF THE INVENTION

Against the above background, the patent applicant, who deeply comprehended shortcomings and drawbacks in the prior art after considering the precious issues, was devoted to inventing a new technique and developing simplified processes for extraction of rice bran after years of research. A rice bran extract composition manufactured to be added into a personal skin-care product for promotions of skin whitening, anti-senescence, anti-inflammation and anti-allergy is a novel and low-cost strategy for skin care. In addition, the present invention alleviates environmental burden imposed by agricultural wastes and lives up to spirit of resource recycling by promoting the value of rice bran and creating extra economic utilization.

The present disclosure is aimed at simplification of extraction processes such as the concentration of a solvent greater than 80%, lower extraction temperature (not more than 55°C) to avoid destroying the efficacy of functional ingredients in rice bran, and extraction time shortened to at least one hour. Moreover, the effects of rice bran extracts are demonstrated through a series of cell assays and the rice bran extracts are manufactured as an easy-to-use product (a rice bran extract composition) for industrial applicability.

To this end, a preparation method of a rice bran extract composition disclosed herein comprises steps as follows: rice bran and a solvent with a concentration more than 80% are mixed for development of a mixed solution and stirred as well as extracted at 40~55°C; the mixed solution is centrifuged or filtered for collections of supernatants or filtrates; the supernatants or filtrates go through a vacuum concentration process for collections of rice bran extracts; the rice bran extracts are treated for preparation of a rice bran extract composition.

In an embodiment of the present disclosure, the ratio of the weight (unit: gram) of rice bran, defatted or non-defatted, to the volume (unit: m1) of the solvent ranges from 1:4 to 1:8.

In an embodiment of the present disclosure, in the solvent the ratio of ethanol or propanol to water is 100~80:0~20 (v/v).

In an embodiment of the present disclosure, the supernatants or filtrates go through a decolorization and purification process through activated charcoal (activated carbon) before the vacuum concentration process which the volume of the supernatants or filtrates is concentrated as 1/18 to 1/22 to get what we called the rice bran extracts.

In an embodiment of the present disclosure, the rice bran extract composition is prepared with an excipient added in a treatment process wherein the excipient includes water, a lipid, an emulgator, a preservative and a cosolvent such that the rice bran extract composition is manufactured as a liquid formulation containing 20~30% rice bran extracts.

In an embodiment of the present disclosure, the rice bran extract composition is prepared with vegetable starch added in a treatment process wherein the ratio of the volume (unit: m1) of rice bran extracts to the weight (unit: gram) of vegetable starch ranges from 1:0.4 to 1:2 for spray drying through which the rice bran extract composition is manufactured as a powder formulation.

In an embodiment of the present disclosure, a process of adding an emulgator, a preservative and a cosolvent is further added before spray drying and the vegetable starch comprise maltodextrin, corn starch, wheat starch, rice starch or tapioca starch.

In an embodiment of the present disclosure, the ratio of the rice bran extracts to the vegetable starch is adjusted by testing the concentration of glucosylceramide inside the rice bran extracts.

A use of the rice bran extract composition for skin care is also offered in the present disclosure wherein the rice bran extract composition is prepared by the preparation method of a rice bran extract composition and features no cytotoxicity, no phototoxicity and no skin irritation but good for skin care such as skin whitening, anti-senescence, anti-inflammation and anti-allergy.

In an embodiment of the present disclosure, an effective amount of the rice bran extracts ranges from 7.8 to 1000 µg/ml.

For all processes with respect to the preparation method of a rice bran extract composition, temperature is a key factor because the effect of a rice bran extract composition (powder formulations or liquid formulations) is worsened if rice bran extracts treated at high temperature for follow-up preparation of the rice bran extract composition, for example, rice bran extracts in a water bath for 0.5-1 hour at 70°C.

The present invention demonstrates the economical use of resources as well as the practice of circular economy. Rice bran as a waste to be disposed is regenerated in the present disclosure, that is, rice bran is recycled as a rice bran extract composition in simplified extraction processes during which a solvent is also recycled in vacuum concentration and reused as the solvent in the present disclosure. Moreover, a rice bran extract composition prepared herein is added into a personal skin-care product for promotions of skin whitening, anti-senescence, anti-inflammation and anti-allergy.

### BRIEF DESCRIPTION OF THE DRAWINGS

The techniques of present invention would be more understandable from the detailed description given herein below and the accompanying figures are provided for better illustration, and thus description and figures are not limitative for present invention, and wherein:
FIG. 1 illustrates test results for cytotoxicity of rice bran extracts in one embodiment.
FIG. 2 illustrates test results for phototoxicity of rice bran extracts in one embodiment.
FIG. 3 illustrates test results for in-vitro skin irritation of rice bran extracts in one embodiment.
FIG. 4 illustrates test results for in-vitro inhibition of tyrosinase due to rice bran extracts in one embodiment.
FIG. 5 illustrates test results for inhibition of melanogenesis due to rice bran extracts in one embodiment.
FIG. 6 illustrates test results for inhibition melanosomes-transferring to keratinocytes due to rice bran extracts in one embodiment.
FIG. 7 illustrates test results for anti-senescence of rice bran extracts in one embodiment.
FIG. 8 illustrates test results for anti-inflammatory of rice bran extracts in one embodiment.
FIG. 9 illustrates test results for anti-allergy of rice bran extracts in one embodiment.
FIG. 10 illustrates test results for inhibition of melanogenesis due to a rice bran extract composition in one embodiment.
FIG. 11 illustrates test results for inhibition melanosomes-transferring to keratinocytes due to a rice bran extract composition in one embodiment.
FIG. 12 illustrates parameters for ions of glucosylceramide (GluCer) and the chromatogram in one embodiment.
FIG. 13 illustrates test results for disappearance of inhibition of melanogenesis due to a rice bran extract composition treated at high temperature in one embodiment.
FIG. 14 illustrates test results for disappearance of inhibition of melanosomes-transferring to keratinocytes due to a rice bran extract composition treated at high temperature in one embodiment.

### DETAILED DESCRIPTION OF THE INVENTION

### [Terminology definition]

The technical and scientific terminologies common in the art of biotech are widely adopted in the patent specification; the definitions of these technical and scientific terminologies are given for clear and consistent understanding of the patent specification as well as claims hereinafter. Other terminologies not defined particularly hereinafter are well known to and commonly understood by persons skilled in the art.

The word like "or" or "and" refers to "and/or" unless otherwise specified. Moreover, the word like "comprise" or "include" is an open-ended term. The descriptions in a previous section refer to general involvement but are not interpreted as restrictions to the subject of the present invention.

The symbol "%" in the patent specification means "weight percentage" unless otherwise specified. The range of a substance A's percentage concentration (for example, "from 10% A to 11% A") should be considered as both the upper and lower limits included (that is, 10%^A's concentration≦11%) unless otherwise specified. The range of a substance B's percentage concentration in which a lower limit is undefined (for example, B less than or under 0.2%) means the lower limit equal to 0 (that is, 0%^B^0.2%). The proportional relationship for ingredients can be transferred to the proportional relationship in "part by weight".

Any numerical value disclosed in the patent specification could imply standard technological measurement deviations (standard deviations) existing such as ± 10%. For ±10%, ±5%, ±2.5% or ±1% relative to a specific numerical value; "about 20%" means 20±2%, 20±1%, 20±0.5% or 20±0.25%.

The term of "effective amount" in the patent specification means a sufficient dose for an effective active substance, which is a dosage given to a receiver for preventing or alleviating one or several diseases or physiological conditions as shown in reliefs and/or alleviations of signs, symptoms, pathologies or other intentional changes in a physiological system.

### [Raw rice bran]

The condition of rice bran in the present disclosure is unrestricted. For example, the condition of rice bran could be untreated or treated (i.e., fermented or defatted); in an embodiment, rice bran in the present disclosure is defatted or non-defatted rice bran. Moreover, the materials used in the present disclosure are commercially available unless otherwise specified.

### [Mixing and extraction process]

A preparation method of a rice bran extract composition in the present disclosure comprises a step to mix rice bran and an 80% solvent for a mixed solution which will be stirred and extracted at 40~55°C; a solvent is characteristic of but not limited to the concentration greater than 80% (w/w) or greater than 80% (v/v), which depends on a type of the solvent.

The ratio of rice bran to a solvent is unrestricted. In addition, the ratio of the weight of rice bran (unit: gram) to the volume of a solvent (unit: ml) ranges from 1:4 to 1:8 preferably, from the view of promoting rice bran extraction efficiency, for example, 1:5.5, 1:6, 1:6.5, 1:7, 1:7.5 or between any above two ratios. The mixing and extraction process can be carried out in batches and the ratio of rice bran to a solvent each time is either fixed or changeable.

Temperature for mixing rice bran and a solvent should be kept not more than or less than 55°C but not limited to a fixed temperature. In addition, temperature for mixing rice bran and a solvent is kept at less than 50°Cfrom the view of promoting rice bran extraction efficiency, for example, 5°C, 10°C, 15°C, 20°C, 25°C, 30°C, 35°C, 40°C, 45°Cor between the above two temperature values and 40~50°Cpreferably. The mixing and extraction process can be carried out in batches and temperature for mixing and extraction each time is either fixed or changeable.

Time for stir and extraction after mixing rice bran and a solvent is unrestricted. In addition, time for stir and extraction should last for at least one hour preferably from the view of promoting rice bran extraction efficiency, for example, 1.5 hours, 2 hours, 2.5 hours, 3 hours, more than 3 hours or between any above two time intervals. The mixing and extraction process can be carried out in batches and time for mixing and extraction each time is either fixed or changeable.

The concentration of a solvent should be kept at more than 80%; a solvent not limited to a specific type is ethanol or propanol with a concentration ranging from 80% to 100% (v/v) preferably from the view of promoting rice bran extraction efficiency, for example, ethanol or propanol with a concentration of 82%, 84%, 86%, 88%, 90%, 92%, 94%, 96%, 98%, 99% or between any above two percentages. The ethanol (propanol) comprises any isomer thereof, for example, n-propanol or isopropanol. That is, the ratio of ethanol or propanol to water is 100~80:0~20 (v/v) preferably, for example, 99:1, 98:2, 96:4, 94:6, 92:8, 90:10, 88:12, 86:14, 84:16, 82:18, or between any above two ratios. The mixing and extraction process can be carried out in batches and the type (concentration) of a solvent for mixing and extraction each time is either fixed or changeable.

In the present disclosure, a toxic solvent such as n-hexane may not be used and could be replaced with another human-friendly and sustainable solvent preferably. Moreover, hot-water extraction may be not used in the present disclosure and could be replaced with solvent extraction in which temperature is kept at not more than 55°C preferably.

### [Mixed-solution separation process]

A preparation method of a rice bran extract composition in the present disclosure comprises a step of centrifuging or filtering a mixed solution, after extraction process, for separating rice bran from a solvent as well as collecting supernatants or filtrates.

In an embodiment, centrifugation through which rice bran is separated from a solvent is not limited to a specific revolution per minute. For filtration through which rice bran is separated from a solvent in an embodiment, the mesh aperture of a device for filtration (for example, filter screen, filter cartridge, etc.) is unrestricted and adjusted properly according to status of separation.

In an embodiment, either the mixing and extraction process or the mixed-solution separation process is carried on in batches, for example, in three batches during which three batches of supernatants or filtrates are collected (and/or combined) for the following processes such as decolorization and purification process (negligible as required) and vacuum concentration process. In an embodiment, supernatants or filtrates may be either extracted and separated from each batch independently or the resulting pellet (residue) may undergo another run (or two) of extraction and separation depending on the extraction efficiency.

### [Decolorization and purification process]

In an embodiment, supernatants or filtrates which have been separated after the mixed-solution separation process are decolored and purified for following vacuum concentration. A material for decolorization and purification are unrestricted but selected from activated charcoal preferably from the view of promoting quality of rice bran extracts.

### [Vacuum concentration process]

A preparation method of a rice bran extract composition in the present disclosure comprises a process of vacuum concentration supernatants or filtrates to make rice bran extracts; a method for vacuum concentration is unrestricted, for example, vacuum distillation, vacuum concentration, etc.

In an embodiment, a solvent is recycled from a vacuum concentration process and the recycled solvent with the concentration more than 80% is reused in the present disclosure. That is, the present disclosure also achieves the effect of saving resources. In the present disclosure, a specific concentration of the recycled solvent is contingent on but not limited to the concentration of a solvent used in the mixing and extraction process.

In an embodiment, the volume of supernatants or filtrates is concentrated as but not limited to 1/18 to 1/22 (for example, 1/19, 1/20, 1/21 or between any above two fractions) based on a vacuum concentration method to get rice bran extracts (a concentrated solution) to be further treated.

In an embodiment, the concentrated solution can be further concentrated for stickier rice bran extracts as appropriate. For example, the volume of supernatants or filtrates is concentrated as 1/23, 1/24 or 1/25 for rice bran extracts (a concentrated solution). In an embodiment, the weight of rice bran extracts accounts for but is not limited to 12~18% (e.g., 13%, 14%, 15%, 16%, 17% or between any above two percentages) compared with the total raw rice bran.

### [Treatment process]

A preparation method of a rice bran extract composition in the present disclosure comprises a treatment process through which rice bran extracts are developed into a composition which is suitable for sale as semi-finished product. To manufacture different types of semi-finished product, such as powder or liquid formulations, proper adjustment of the rice bran extract composition may be required.

In an embodiment for a liquid formulation prepared from a rice bran extract, a rice bran extract composition is manufactured with an excipient added into rice bran extracts in a treatment process wherein the excipient includes water, a lipid, an emulgator, a preservative and a cosolvent and the final composition contains 20~30% of rice bran extracts (e.g., 22%, 24%, 26%, 28% or between any above two percentages).

The content (specific type) of water (a lipid, an emulgator, a preservative or a cosolvent) inside the excipient is unrestricted and properly adjusted according to status of a rice bran extract composition as a final product.

In an embodiment for a powder formulation prepared with a rice bran extract, a rice bran extract composition is manufactured by mixing vegetable starch with rice bran extracts in a spray drying process wherein the ratio of the volume of rice bran extracts (unit: ml) to the weight of vegetable starch (unit: gram) ranges from 1:0.4 to 1:2 (e.g., 1:0.5, 1:0.6, 1:0.7, 1:0.8, 1:0.9, 1:1, 1:1.1, 1:1.2, 1:1.3, 1:1.4, 1:1.5, 1:1.6, 1:1.7, 1:1.8, 1:1.9 or between any above two ratios).

In an embodiment, the vegetable starch is not limited to a specific type and comprise maltodextrin, corn starch, wheat starch, rice starch or tapioca starch from the view of promoting quality of a powder formulation.

In an embodiment, a further step for addition of an emulgator, a preservative or a cosolvent before spray drying promotes homogeneous mixing of rice bran extracts (a concentrated solution) for follow-up spray drying with starch. In addition, the type or content of an ingredient such as emulgator, preservative or cosolvent is unrestricted and properly adjusted according to status of a rice bran extract composition as a final product.

In an embodiment, a liquid or powder formulation of a rice bran extract composition made of rice bran extracts (a concentrated solution) proves effective in reproducibility through a cell assay.

### [Quality control]

Quality discrepancy of rice bran as a natural product under identical preparation conditions is blamed on multiple factors such as area for growth of rice and different weather every year. To inspect discrepancy between batches, we designate marker constituents through which a ratio of rice bran extracts (a concentrated solution) to vegetable starch for spray drying is adjusted.

The marker constituents include oryzanol, vitamin E, squalene or glucosyceramide (GluCer). Due to the minimal amount and identifiable properties of glucosylceramide, it is recommended that the concentration of glucosyceramide inside rice bran extracts is checked for changing the ratio of rice bran extracts to vegetable starch.

### [Skin care purpose]

The present disclosure is also aimed at providing rice bran extracts for skin care. For that matter, rice bran extracts prepared hereinbefore is characteristic of no cytotoxicity, no phototoxicity, no skin irritation but good for skin care such as skin whitening, anti-senescence, anti-inflammation and anti-allergy.

In an embodiment, safeties of rice bran extracts for cell viability/cytotoxicity, phototoxicity and in-vitro skin irritation are authenticated in cell assays in which efficacy of rice bran extracts for skin-whitening, anti-senescence, anti-inflammatory and anti-allergy are also checked.

In an embodiment, the effective amount of rice bran extracts in a rice bran extract composition ranges from 7.8 to 1000µg/ml, for example, 15.6µg/ml, 31.2µg/ml, 62.5µg/ml, 125µg/ml, 250µg/ml, 500µg/ml or between any above two values.

A preparation method of a rice bran extract composition is explained in following several embodiments/comparisons which are not regarded as examples to limit the present invention; any modification or change without departing from the spirit and scope of the present invention can be made by a person skilled in the art. Moreover, all materials hereinafter are commercially available.

### Experiment 1: Preparation of rice bran extracts

In Experiment 1, 100g rice bran is added into a solvent (a mixed solution containing 400mL ethanol and 50mL water for development of 89% ethanol), stirred for one hour at 42°C and centrifuged for collections of supernatants. The extraction process is further repeated twice for collections of three batches of supernatants totally. 1000~1200mL supernatants collected totally are mixed for follow-up activated charcoal treatment as well as vacuum concentration after which 15g sticky rice bran extracts (extraction rate=15%) are derived.

### Experiment 2: Cell viability/cytotoxicity assay

In Experiment 2, 8×10³ L929 fibroblasts of a mouse are inoculated into a 96-well plate for cell attachment in 24 hours and treated with different-concentration rice bran extracts for 24 to 48 hours. With broths removed, the MTS reagent is added and reacts with fibroblasts for 2 to 4 hours after which the absorbance at 490 nm is checked with a micro-plate reader. Fibroblasts in the NC (negative control) group are treated with broths (1% DMSO) in which no rice bran extract is added for fibroblasts alive; fibroblasts in the PC (positive control) group are treated with broths (5% DMSO) for no fibroblast alive. For comparisons with another group in the assay, the cell viability of the NC group based on the absorbance is set to 100%. The cell viability up to 80% is taken as a reference for the safe concentration of a sample.

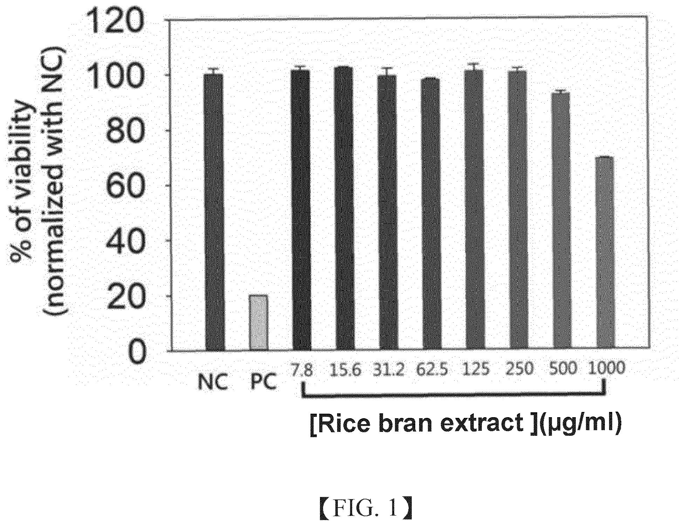

Referring to FIG. 1, which illustrates test results for cytotoxicity of rice bran extracts. As shown in FIG. 1, the cell viabilities for 500µg/mL and 1000µg/mL rice bran extracts prepared herein is greater than 90% and 70%, respectively.

### Experiments: Phototoxicity assay

According to the OECD test guideline No. 432, 1×10⁴ 3T3 cells of a mouse are inoculated into two 96-well plates and cultured for 24 hours. Then, rice bran extracts and CPZ (Chlorpromazine for the positive control group) are added into the two 96-well plates, respectively. In each group, there are eight concentrations to be checked. After dark culture in one hour at 37°C, the 3T3 cells in one 96-well plate are exposed to UVA (5J/cm²) for 1/2 to 1 hour but the 3T3 cells in the other 96-well plate are still kept at dark culture. With new broths replacing original ones, the 3T3 cells are cultured for another 18 hours. Neural red stains are added for a reaction in three hours after which 3T3 cells rinsed with phosphate buffers are extracted based on neural red stains inside 3T3 cells and the absorbance at 540 nm is checked with a micro-plate reader (Note: a high absorbance means a high cell viability). The curves for concentrations of determinands vs. cell viabilities are drawn for estimation of IC50. It can be seen from the formula of photo irritation factor (PIF), [IC50(-UV)/IC50(+UV)] ^6, that phototoxicity risk exists.

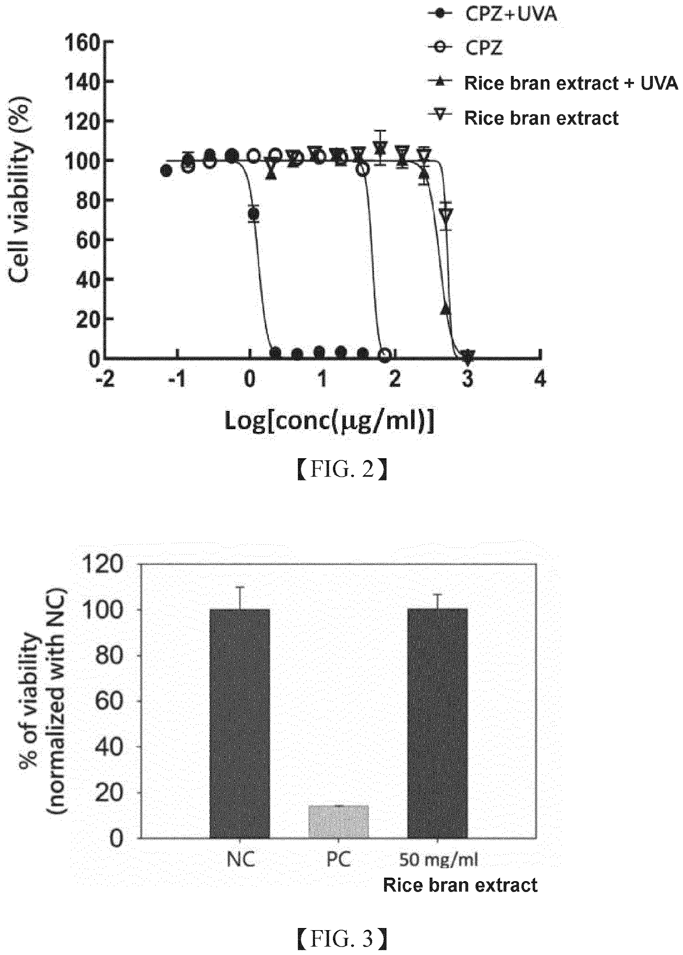

Referring to FIG. 2, which illustrates test results for phototoxicity of rice bran extracts. A left shift of the curve for cell viability after expose to UVA means existence of phototoxicity. As shown in FIG. 2, the PIF of rice bran extracts prepared herein ranges from 1.28 to 1.67 (Note: no curves are derived for some batches). It can be seen from test results that the phototoxicity risk of rice bran extracts (PIF lower than 2) in the present disclosure is very low in contrast to the PC group (CPZ greater than 31).

### Experiment 4: In-vitro skin irritation assay

The assay is conducted on in-vitro reconstructed human epidermis (RhE), which mimics the upper parts of human skin using non-transformed human-derived epidermal keratinocytes, according to the OECD test guideline No. 439. With the concentration of a determinand (the maximum concentration of a determinand to be added into a product or the concentration of a determinand 2~3 times higher than a normal concentration) selected for the assay, the determinand is coated on and reacts with the apical surface of mimic skin for 15 minutes at room temperature. On the other hand, 5% SDS (PC group) and a solvent (mineral oil; NC group, which is used to prepare rice bran extracts for assay) are also coated, respectively. With samples removed from mimic skin, the apical surface is rinsed with phosphate buffers three times and added by the MTS reagent for a reaction in 24 hours after which the absorbance at 490 nm is checked with a micro-plate reader. The absorbance for the NC group is set to the cell viability of 100%; the cell viability for the PC group is less than 50%; the evidence that the cell viability greater than 50% is checked at a sample is regarded as no skin irritation.

Referring to FIG. 3, which illustrates test results of rice bran extracts in the in-vitro skin irritation assay. As shown in FIG. 3, no skin irritation is attributed to rice bran extracts (concentration=50mg/mL) prepared herein.

### Experiment 5: Skin whitening test (assay for in-vitro inhibition of tyrosinase)

Samples with different concentrations in buffers are added into a 96-well plate; kojic acid (final concentration=6.25µg/ml) and buffers with 2% DMSO but no determinand are included in assay as the PC group and the NC group, respectively. 125U mushroom tyrosinase is added into the 96-well plate for a five-minute reaction at room temperature after which L-tyrosine as a substrate is added for checking any change in the absorbance at 475nm per minute with a micro-plate reader in 30 minutes. The absorbance will grow over time in the case of activity of tyrosinase uninhibited; on the other hand, activity of tyrosinase inhibited is accompanied by the growth of absorbance weakened. The activity of tyrosinase is compared by checking changes in the absorbance per unit time from 0 to 15 minutes.

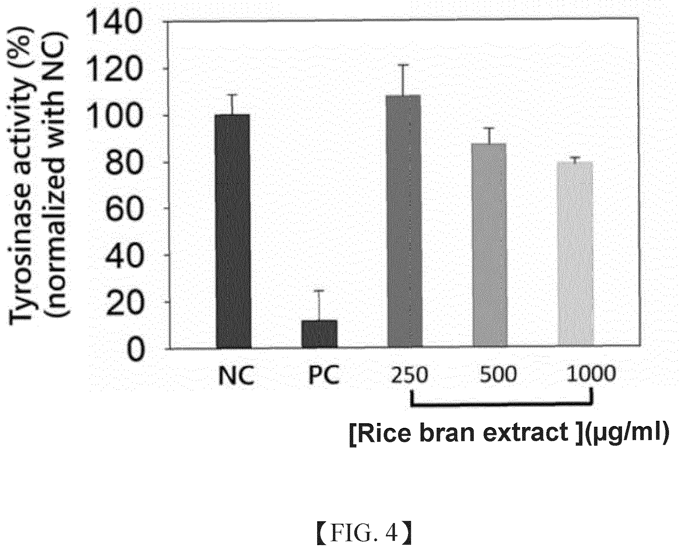

Referring to FIG. 4, which illustrates test results of the assay for in-vitro inhibition of tyrosinase. As shown in FIG. 4, there is a positive correlation between the activity of tyrosinase inhibited due to rice bran extracts prepared herein and the concentration.

### Experiment 6: Skin whitening test (assay for anti-melanogenesis in B16F10 cells)

2×10⁴ melanoma cells (B 16/F10) are inoculated into a 6-well plate for cell attachments in 24 hours. With broths removed, 100ng/mL melanocyte stimulating hormone (α-MSH) is added into the 6-well plate for a 30-minute reaction. Then, equivoluminal broths featuring the concentration twice higher than the concentration of extracts are added: in broths of the NC group containing no rice bran extracts; in broths of the PC group containing kojic acid, whose final working concentration in assay is 0.5mg/ml. Melanoma cells of the base-line group are not treated with α-MSH. With broths removed after cultivation in 72 hours, melanoma cells are rinsed with phosphate buffers and added by 0.25% trypsin such that cells desorbed are collected through centrifugation. Melanoma cells collected are further rinsed with sterile phosphate buffers two or three times; precipitated cell in which 100ul M-PER cell lysis buffers are added are broken up and centrifuged to obtain supernatants for follow-up quantification of protein concentration. The precipitates then proceed to measurement of melanin, that is, the 1N NaOH solution is added into and reacts with precipitates for two hours at 80°C. After centrifugation, supernatants are collected in a 96-well plate for checking the absorbance at 405 nm with a micro-plate reader (Note: the weaker absorbance is detected, the less melanin exists). Before comparisons of data between an experimental group, the PC group and the NC group, the absorbance for melanin is standardized with autologous protein concentration. The standardized content of melanin for the NC group is set to 100%.

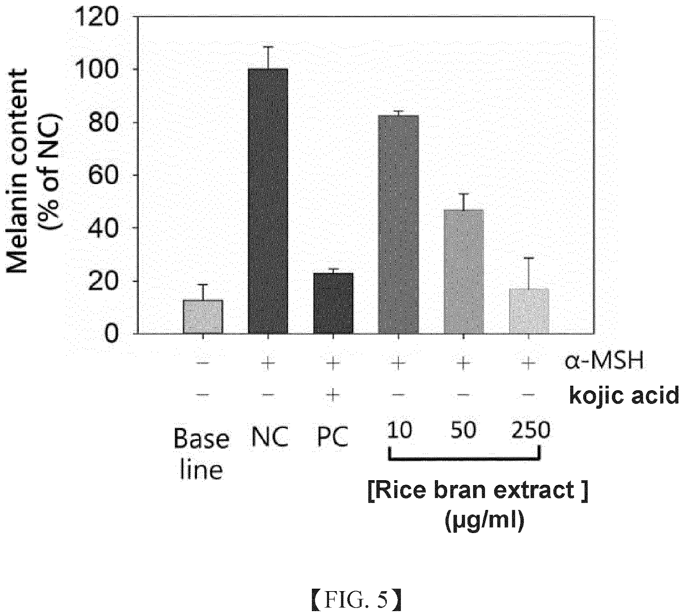

Referring to FIG. 5, which illustrates test results for inhibition of melanogenesis due to rice bran extracts. As shown in FIG. 5, melanogenesis is inhibited by 50µg/mL rice bran extracts prepared herein and the effect for anti-melanogenesis of 250µg/mL rice bran extracts is better than that of 500µg/mL kojic acid.

### Experiment 7: Skin whitening test (assay for anti-melanosomes-transferring to keratinocytes)

As shown in skin whitening tests in Experiments 5 and 6, the test results of the in-vitro assay different from those of the assay based on B 16F10 cells may be attributed to multiple physiological mechanisms of rice bran extracts for skin whitening. Accordingly, another assay based on keratinocytes is conducted in the present disclosure for promotion of skin whitening and review of a skin whitening mechanism of rice bran extracts prepared herein from another point of view.

Melanin is a biochrome with strong antioxidant capacity. When keratinocytes are stimulated by UV, they endocytose melanosomes, secreted by melanocytes, to surround their nuclei for protect genetic material such as DNA from mutations. The normal protective mechanism is one factor of skin darkening. Endocytosis of melanosomes by keratinocytes will be promoted due to stimulation of a low growth factor, i.e., starvation. Accordingly, with fluorescent beads similar in size to melanosomes selected to simulate melanosomes, the evidences for inhibition of melanosomes endocytosed by keratinocytes due to rice bran extracts and the mechanism with respect to skin darkening are explored.

In Experiment 7,1×10⁴ primary keratinocytes are inoculated into a 24-well plate and cultured by broths containing 10% serums (growth factor) for cell attachment. After which broths are removed and replaced by fresh broths containing 0~2% serums and rice bran extracts for co-cultivation another 4 to 6 hours. Then, red fluorescent microbeads (0.5 µm) similar in size to melanosomes are added for a reaction in two hours. After red fluorescent microbeads not endocytosed by keratinocytes are removed, keratinocytes are fixed with a 4% formalin solution and cell nuclei are stained by DAPI. The count of red fluorescent microbeads endocytosed by 50 keratinocytes is checked under a fluorescence microscope (ex/em: 580/605 nm) for calculating a mean value: "0~5": 0; "6~10": 1 point; "11-15": 2 points; "16~20": 3 points; "21-25": 4 points; ">26": 5 points. Keratinocytes in the PC group (blank control group) are cultivated under the growth factor concentration of 10% (for example, keratinocytes nutritionally adequate inside 10% serums for no endocytosis); keratinocytes in the NC group are cultivated in 0~2% serums as non-extracts (for example, 2% serums for endocytosis of melanosomes by keratinocytes stimulated by starvation).

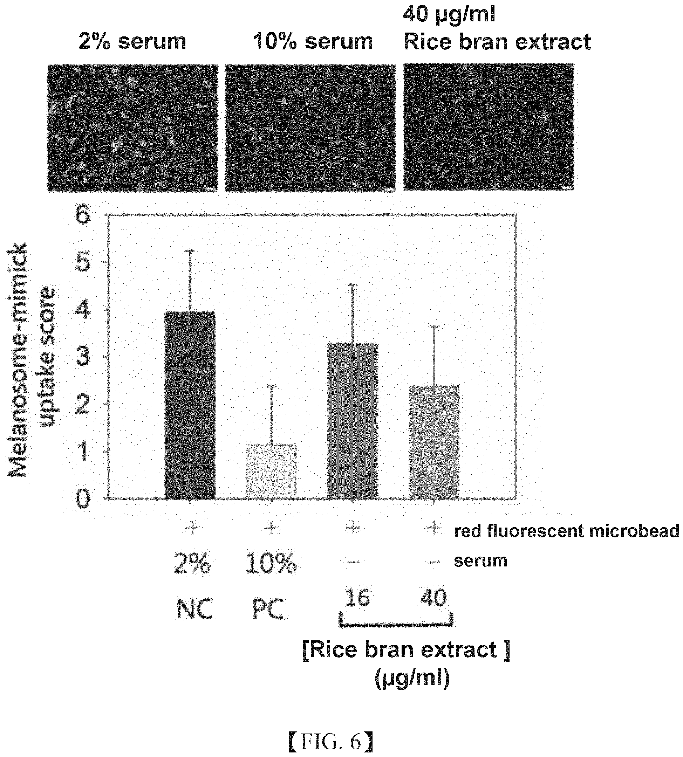

Referring to FIG. 6, which illustrates test results for anti-melanosomes-transferring to keratinocytes due to rice bran extracts. As shown in FIG. 6, a positive correlation exists between concentrations of rice bran extracts ranging from 16 to 40µg/mL and inhibition of melanosomes endocytosed by keratinocytes.

### Example 8: Anti-senescence assay

5×10⁴ fibroblasts are inoculated into a 6-well plate for cultivation overnight. After broths are removed, fresh broths (free of serums) containing 8mM hydroxyurea and rice bran extracts are added and co-cultures for 24 hours. With broths containing serums replacing original ones for further cultivation in 24 hours, fibroblasts are rinsed with phosphate buffers twice, fixed with a 3.7% formalin solution and stained for β-galactosidase. Fibroblasts in the base-line group are not treated with hydroxyurea; fibroblasts in the NC group for which rice bran extracts are not added are treated with hydroxyurea. Hydroxyurea induces senescence and promotes SA-β-galactosidase inside fibroblasts which turn blue after staining, that is, the low percentage of fibroblasts turning blue means good anti-senescence of fibroblasts. In Example 8, the percentage of fibroblasts turning blue among 100 fibroblasts for 3~5 fields under an optical microscope is checked.

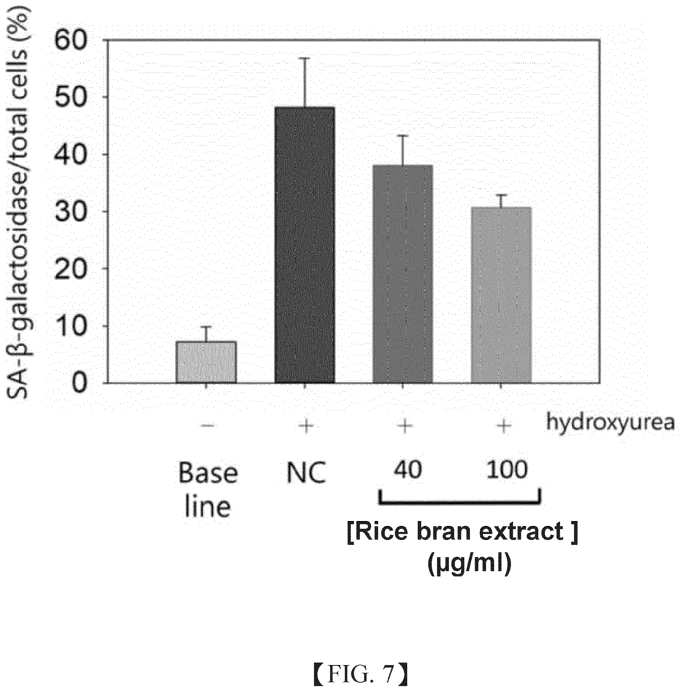

Referring to FIG. 7, which illustrates test results for anti-senescence of rice bran extracts. As shown in FIG. 7, the percentage of fibroblasts turning blue due to rice bran extracts prepared herein reduces and is positively correlated with the concentration of rice bran extracts which proves effective in anti-senescence.

### Experiment 9: Anti-inflammatory assay

2×10⁴ macrophages (RAW264.7) are inoculated into a 96-well plate. After completion of cell attachment, original broths are removed and replaced by new broths containing 250ng/mL lipopolysaccharide (LPS) and rice bran extracts. The NC group is characteristic of broths free of rice bran extracts (without nitroarginine) compared with broths of the PC group including 1mM nitroarginine (LNNA). For that matter, nitrites are induced by LPS but inhibited by LNNA. With macrophages cultivated for 24 hours, nitrites produced in broths are tested with the Griess reagent. Nitric oxide (NO) is one of regulatory factors of intracellular inflammation and the lower NO content, the lower inflammation response. Nitrite, as an indicator of NO, its production should be inhibited for better anti-inflammation effect.

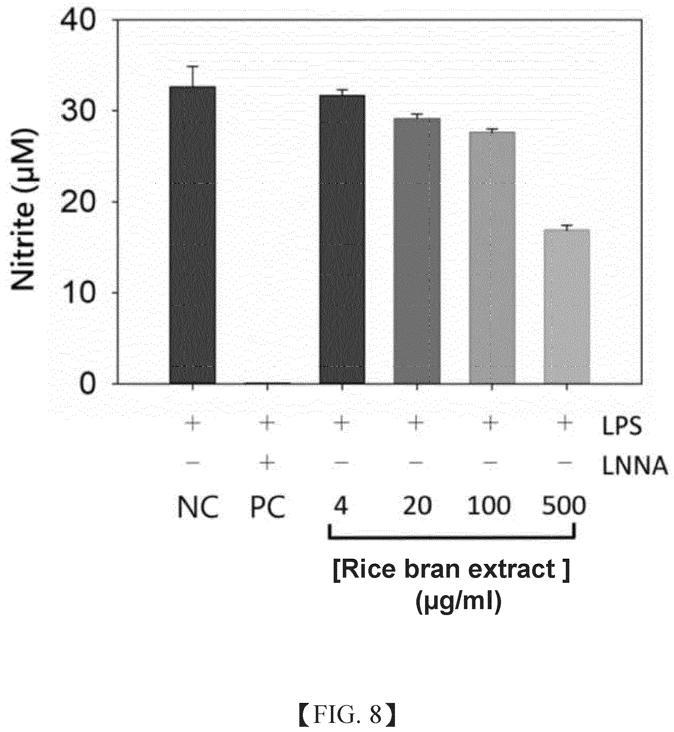

Referring to FIG. 8, which illustrates test results for anti-inflammation of rice bran extracts. As shown in FIG. 8, nitrites are significantly inhibited by 500µg/mL rice bran extracts prepared herein for a good anti-inflammation effect.

### Experiment 10: Anti-allergy assay

3×10⁵ basophils (RBL-2H3) are inoculated into and cultivated in a 24-well plate for 24 hours. With original broths removed, new broths with rice bran extracts are added into basophils for cultivation in 4 to 6 hours. Then, 500ng/mL medication A23187 is added for stimulation in 30 minutes and activity of β-hexosaminidase for collected supernatants is checked. Basophils of the base-line group are treated with neither ketotifen nor A23187; the NC group features broths free of rice bran extracts (basophils in which no ketotifen is given but A23178 is added for inducing enzyme activity); basophils of the PC group are provided with 0.3mM ketotifen and then A23178 for inducing enzyme activity. After 30µL culture supernatants react with a 50µL substrate solution (p-nitrophenyl N-acetyl-Dglucosamine; concentration=1.3 mg/mL) for one hour at 37°C, a 0.5M NaOH solution is mixed for terminating reactions and checking the absorbance at 405nm with a micro-plate reader. The weakened absorbance means low activity of β-hexosaminidase that will relieve allergies. A23187 induces RBL-2H3 to degranulate for release of lots of inflammatory mediators; among them β-hexosaminidase is usually taken as a biochemical criterion for checking allergy.

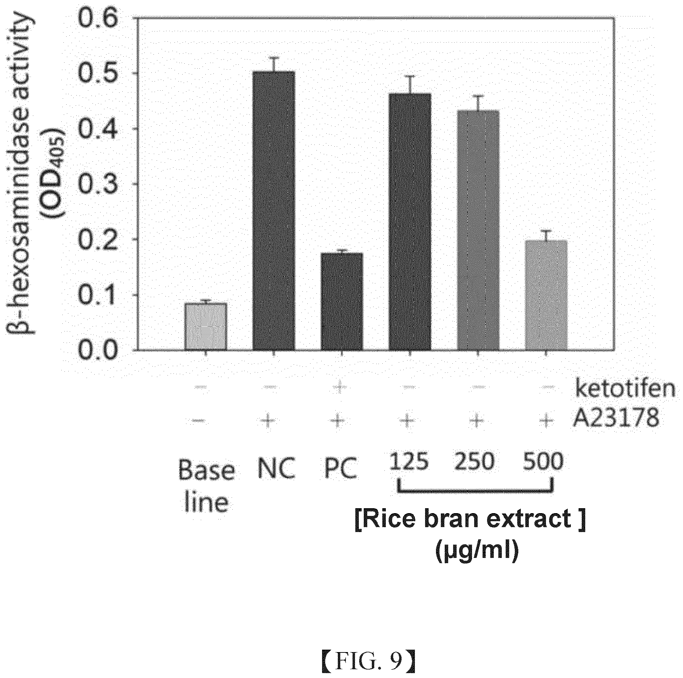

Referring to FIG. 9, which illustrates test results for anti-allergy of rice bran extracts. As shown in FIG. 9, activity of β-hexosaminidase is significantly inhibited by 500µg/mL rice bran extracts prepared herein for a good anti-allergy effect.

### Experiment 11: Treatment process for preparation of liquid formulations

Experiment 11 conducted in the present disclosure consists of two parts, oil phase and water phase.

Oil phase: Rice bran extracts, an emulgator, glycerin and a preservative are heated to 50°C for mixing.

Water phase: Water, a preservative and a cosolvent are heated to 50°C for mixing.

The substances in the oil phase are poured into and mixed with substances in the water phase for preparing a rice bran extract composition (liquid formulations) containing 20~30% rice bran extracts.

### Experiment 12: Treatment process for preparation of powder formulations

It is generally considered that powder materials are easily weighted or blended during preparation of a skin care product. For industrial applicability of the preparation method of a rice bran extract composition in the present disclosure, the scale of extractions is magnified hundredfold in Experiment 12 and rice bran extracts are further manufactured as powder formulations.

10kg rice bran and a solvent (a mixed solution containing 60L ethanol and 7.5L water) added are stirred for one hour at 42°C. With the mixture centrifuged, supernatants are collected and rice bran is filtered; then, another solvent (a mixed solution containing 50L ethanol and 6.25L water) is added again for extracting supernatants once more. All supernatants collected are combined and treated with activated charcoal and finalized to a total volume of supernatant around 100L. The supernatants go through a vacuum concentration process through which a 5L concentrated solution (rice bran extracts) is derived. Then, a rice bran extract composition (powder formulations) is prepared with a preservative Spectrastat^{™} E, butylene glycol added into the concentrated solution and spray-dried with 3000g maltodextrin.

### Experiment 13: Skin whitening effect of a rice bran extract composition

As shown in previous experiments, rice bran extracts have proven effective in skin whitening. In Experiment 13, a rice bran extract composition (powder formulations) prepared herein is also tested for its skin whitening effect as indicated by test results for multiple cells in those previous experiments.

The test conducted in Experiment 13 is identical to tests presented in Experiment 6, assay for anti-melanogensis in B16F10 cells, and Experiment 7, assay for anti-melanosomes-transferring to keratinocytes, wherein rice bran extracts are replaced with a rice bran extract composition (powder formulations).

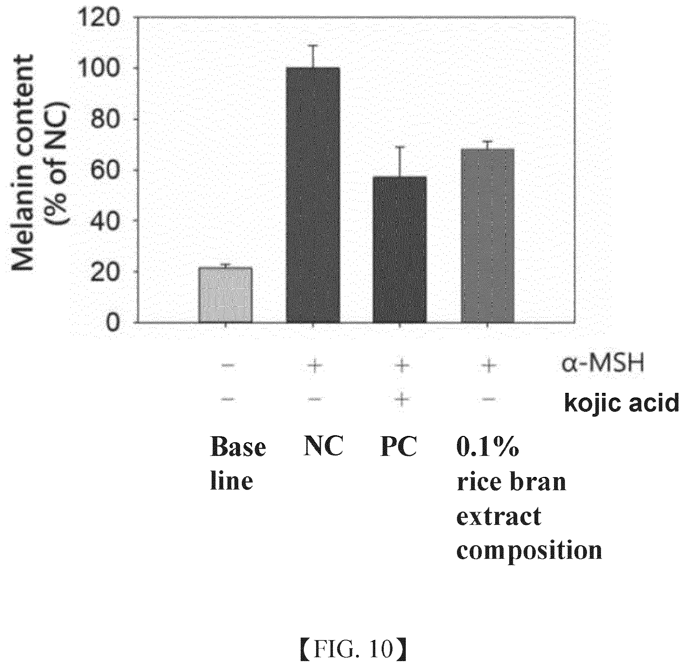

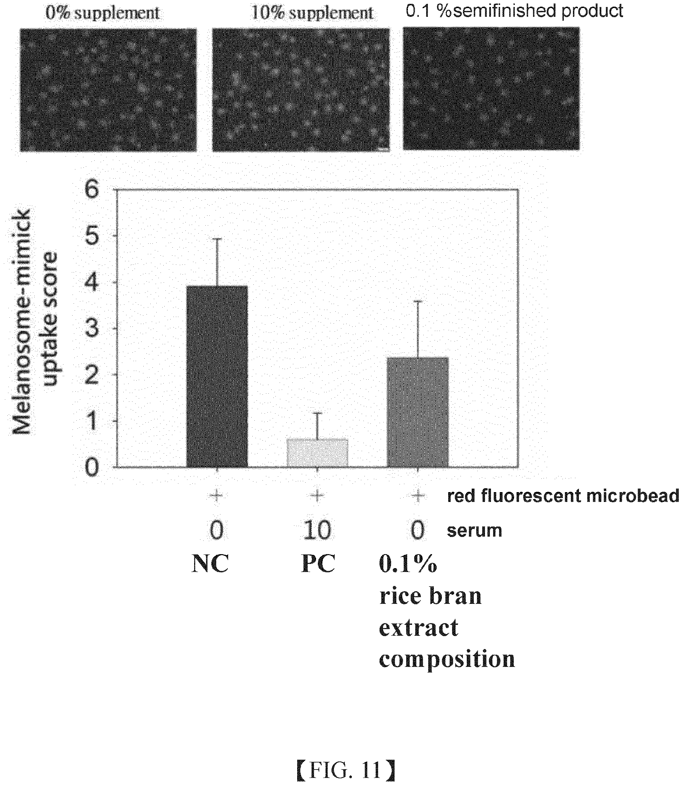

Referring to FIGS. 10 and 11, which illustrate test results for inhibition of melanogenesis and melanosomes-transferring to keratinocytes due to a rice bran extract composition, respectively. As shown in FIGS. 10, the effects for inhibition of melanogenesis and inhibition of melanosomes endocytosed by keratinocytes due to a rice bran extract composition (concentration=0.1%) prepared herein are comparable to those of 500µg/mL kojic acid. Experiment 14: Marker constituents for a rice bran extract composition

Marker constituents are used in detecting any discrepancy during extractions from batch to batch and taken as a criterion with which the ratio of a concentrated solution (rice bran extracts) to vegetable starch during spray drying is adjusted. In Experiment 14, the 0.25-0.5g rice bran extract composition which is well mixed with 10mL water is diluted with 100% methanol by a 20-fold to 50-fold dilution and filtered. For quantitative and qualitative analyses of glucosylceramide (GluCer), 5uL filtrate is injected into LC-MSMS (Note: column: Agilent Poroshell 120 EC-C18 4.6*50mm 2.7-Micron; mobile phase: 100% methanol containing 0.5~5mM ammonium acetate; flow rate: 1mL/min; temperature: 40°C). If necessary, galactosylceramide as an internal standard can be added.

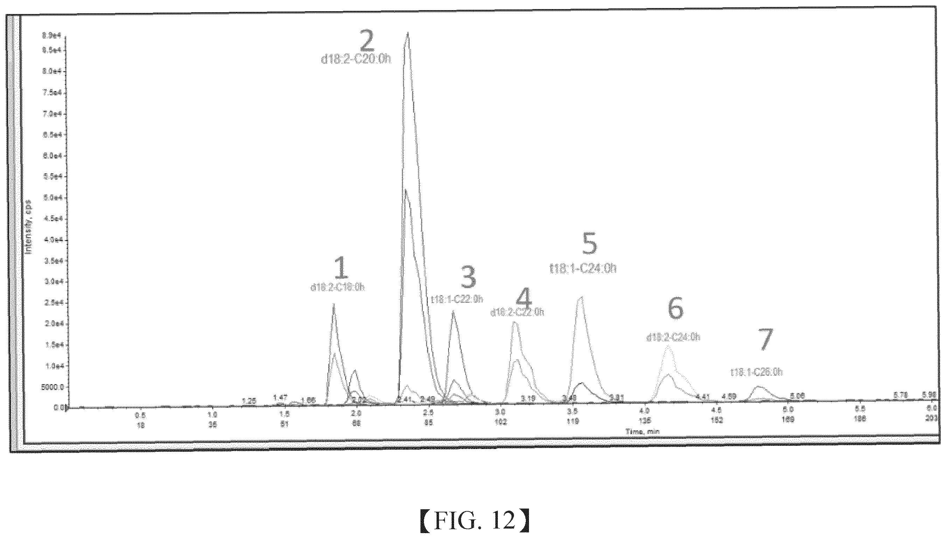

Referring to Table 1 and FIG. 12, which illustrate parameters for ions of GluCer and the chromatogram, respectively. The concentration of GluCer inside a rice bran extract composition prepared herein ranges from 0.01 to 0.03%.

**[Table 1] Parameters for ions of GluCer**

| | | Precursor ion, Q1 | Product ion, Q3 | | |
|---|---|---|---|---|---|
| Category | RT time | [M+H]⁺ | Sphingoid base [B+H-2H₂O]⁺ | [M+H-180]⁺ | Peak |
| d18:2-C16:0h | 1.48 | 714.8 | 262.2 | 534.2 | |
| d18:2-C18:0h | 1.85 | 742.8 | 262.2 | 562.2 | 1 |
| d18:2-C19:0h | 2.11 | 756.8 | 262.2 | 576.2 | |
| d18:2-C20:0h | 2.38 | 770.8 | 262.2 | 590.4 | 2 |
| d18:2-C22:0h | 3.12 | 798.8 | 262.2 | 618.5 | 4 |
| d18:2-C24:0h | 4.18 | 826.8 | 262.2 | 646.5 | 6 |
| t18:1-C22:0h | 2.70 | 816.8 | 262.2 | 636.5 | 3 |
| t18:1-C24:0h | 3.57 | 844.8 | 280.2 | 664.5 | 5 |
| t18:1-C26:0h | 4.83 | 872.8 | 280.2 | 692.5 | 7 |
| d18:1-C20:0h | 2.37 | 772.6 | 264.2 | 592.5 | |

### Experiment 15: Importance of temperature

The importance of specific temperature for a preparation method of a rice bran extract composition is explained in Experiment 15. 4kg rice bran and a solvent (a mixed solution containing 24L ethanol and 3L water) added are stirred for one hour at 42°C. With the mixture centrifuged, supernatants are collected and rice bran is filtered; then, another solvent (a mixed solution containing 20L ethanol and 2.5L water) is added again for extracting supernatants once more. All supernatants collected are combined and treated with activated charcoal and finalized to a total volume of supernatant around 40L. The supernatants go through a vacuum concentration process from which a 1.9L concentrated solution (rice bran extracts) is derived; moreover, a preservative Spectrastat^{™} E, butylene glycol are added into the concentrated solution and spray-dried with 2500g maltodextrin. The concentration of GluCer checked with LC-MSMS is 0.019% which is designated for the normal group.

10kg rice bran and a solvent (a mixed solution containing 60L ethanol and 7.5L water) added are stirred for one hour at 42°C. With the mixture centrifuged, supernatants are collected and rice bran is filtered; then, another solvent (a mixed solution containing 50L ethanol and 6.25L water) is added again for extracting supernatants once more. All supernatants collected are combined and treated with activated charcoal and finalized to a total volume of supernatant around 105L. The supernatants go through a vacuum concentration process from which a 5L concentrated solution (rice bran extracts) is derived; moreover, a preservative Spectrastat^{™} E and butylene glycol are added into the concentrated solution and from which 2.5L concentrated solution is taken and immersed in the water bath for one hour at 70°C after which the concentrated solution is spray-dried with 3000g maltodextrin. The concentration of GluCer checked with LC-MSMS is 0.029% which is designated for the high-temperature treatment group. Theoretically, the good test results of a cell assay should be credited to the group with higher-concentration of GluCer, which was used as a marker constituents for a rice bran extract composition. The higher GluCer the more skin-whitening components within the rice bran extracts.

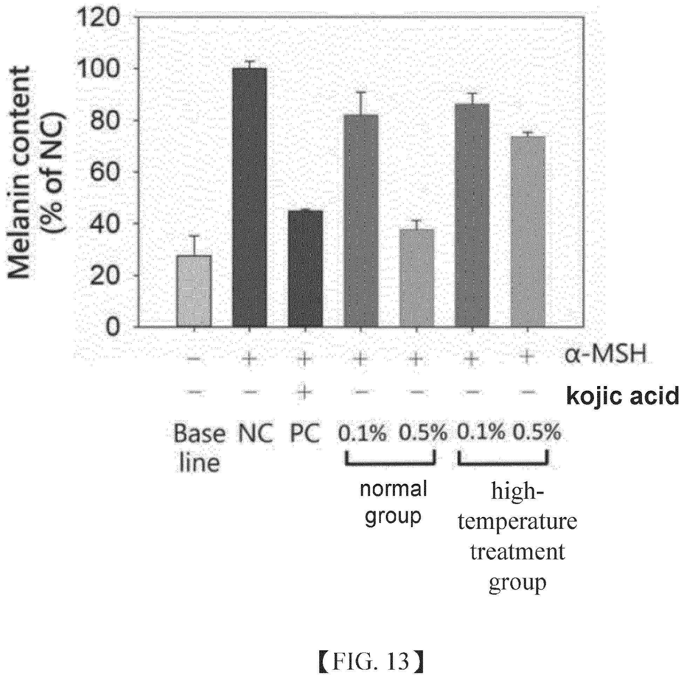

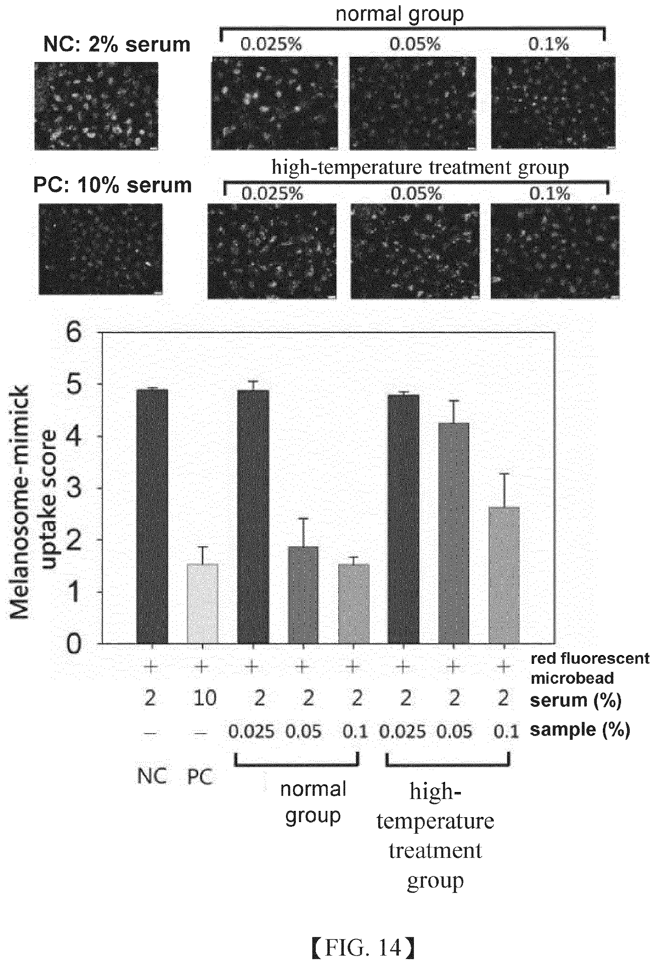

In Experiment 15, similar to Experiments 6 and 7 in the test method, a rice bran extract composition designated for the normal group or the high-temperature treatment group, mentioned above, acts as a substitute for rice bran extracts. Referring to FIGS. 13 and 14, which illustrate test results for inhibition of melanogenesis and melanosomes-transferring to keratinocytes due to a rice bran extract composition (normal group or high-temperature treatment group), respectively. As shown in FIGS. 13 and 14, the level for inhibition of melanogenesis (or melanosomes-transferring to keratinocytes) of the high-temperature group, even though containing higher-concentration GluCer (0.029%), is only half of the level of a rice bran extract composition in the normal group (0.019% GluCer), that is, temperature is crucial to the effect of a rice bran extract composition on skin whitening. Accordingly, temperature for the preparation method of a rice bran extract composition should be less than 55°C and between 40 and 50°C preferably for a good effect of skin whitening.

In summary, rice bran rich in healthcare elements but usually abandoned are recycled and manufactured as a rice bran extract composition through simple processes in the present disclosure and a solvent treated in a vacuum concentration process is also recycled and reused as the solvent during preparation of the present invention, that is, the present invention which economizes resources and effectuates recycling economy is categorized into the scope of green science and technology. Moreover, a rice bran extract composition herein proves effective in skin whitening, anti-senescence, anti-inflammation and anti-allergy.

The embodiments disclosed hereinbefore describe the technological philosophy and features of the present invention and are implemented by persons skilled in the art and familiarizing themselves with the content of the present disclosure but not regarded as examples to limit the scope of claims hereinafter, that is, any equivalent modification or change based on the spirit disclosed herein should be incorporated into claims of the present invention.

Accordingly, it is to be understood that the embodiments of the invention herein described are merely illustrative of the application of the principles of the invention. Reference herein to details of the illustrated embodiments is not intended to limit the scope of the claims, which themselves recite those features regarded as essential to the invention.

## Claims

1. A preparation method of a rice bran extract composition, comprising steps as follows:
rice bran and a solvent with a concentration more than 80% are mixed for development of a mixed solution and stirred as well as extracted at 40~55°C;
the mixed solution is centrifuged or filtered for collections of supernatants or filtrates;
the supernatants or filtrates go through a vacuum concentration process for collections of rice bran extracts; and
the rice bran extracts are treated for preparation of a rice bran extract composition.

2. The preparation method of a rice bran extract composition as claimed in claim 1 wherein the rice bran is defatted or non-defatted; the ratio of the weight (unit: gram) of the rice bran, to the volume (unit: ml) of the solvent ranges from 1:4 to 1:8.

3. The preparation method of a rice bran extract composition as claimed in claim 1 wherein the ratio of ethanol or propanol in the solvent to water in the solvent is 100~80:0~20 (v/v).

4. The preparation method of a rice bran extract composition as claimed in claim 1 wherein the supernatants or filtrates go through a decolorization and purification process through activated charcoal before the vacuum concentration process based on vacuum distillation with which the volume of the supernatants or filtrates is concentrated as 1/18 to 1/22 for preparation of the rice bran extracts.

5. The preparation method of a rice bran extract composition as claimed in claim 1 wherein: the rice bran extract composition is prepared with an excipient added in a treatment process; the excipient includes water, a lipid, an emulgator, a preservative and a cosolvent such that the rice bran extract composition is manufactured as a liquid formulation containing 20~30% rice bran extracts.

6. The preparation method of a rice bran extract composition as claimed in claim 1 wherein: the rice bran extract composition is prepared with vegetable starch added in a treatment process; the ratio of the volume (unit: ml) of rice bran extracts to the weight (unit: gram) of the vegetable starch ranges from 1:0.4 to 1:2 for spray drying through which the rice bran extract composition is manufactured as the powder formulation.

7. The preparation method of a rice bran extract composition as claimed in claim 6 wherein: a process of adding an emulgator, a preservative and a cosolvent is further added before spray drying; the vegetable starch comprise maltodextrin, corn starch, wheat starch, rice starch or tapioca starch.

8. The preparation method of a rice bran extract composition as claimed in claim 6 wherein the ratio of the rice bran extracts to the vegetable starch is adjusted by testing the concentration of glucosylceramide inside the rice bran extracts.

9. A use of the rice bran extract composition for skin care wherein the rice bran extract composition is prepared by the preparation method as claimed in claim 1 and the rice bran extract composition is characteristic of no cytotoxicity, no phototoxicity, no skin irritation but good for skin care such as skin whitening, anti-senescence, anti-inflammation and anti-allergy.

10. The use of the rice bran extract composition for skin care as claimed in claim 9 wherein an effective amount of the rice bran extracts ranges from 7.8 to 1000 µg/ml.
